# EUROPÄISCHE PATENTSCHRIFT

(11) **EP 4 385 429 B1**
(45) Veröffentlichungstag und Bekanntmachung des Hinweises auf die Patenterteilung: **26.11.2025**
(21) Anmeldenummer: 23217001.9
(22) Anmeldetag: 15.12.2023
(51) Int. Cl.: A61B 17/22, A61B 17/32, B06B 1/06, A61B 17/00

(54) **HALTEVORRICHTUNG FÜR EINE LITHOTRIPSIEVORRICHTUNG ZUM ZERTRÜMMERN VON KÖRPERSTEINEN UND LITHOTRIPSIEVORRICHTUNG**
HOLDING DEVICE FOR A LITHOTRIPSY DEVICE FOR DISINTEGRATING BODY STONES AND LITHOTRIPSY DEVICE
DISPOSITIF DE MAINTIEN POUR UN DISPOSITIF DE LITHOTRIPSIE DESTINÉ À LA DESTRUCTION DE CALCULS CORPORELS ET DISPOSITIF DE LITHOTRIPSIE

(30) Priorität: 15.12.2022 DE 102022133520
(43) Veröffentlichungstag der Anmeldung: 19.06.2024
(73) Patentinhaber: Karl Storz SE & Co. KG, 78532 Tuttlingen (DE)
(72) Erfinder: GLÖGGLER, Bernhard, 78532 Tuttlingen (DE)

(56) Entgegenhaltungen:
- DE-A1- 102022 109 138
- US-A- 5 397 293
- US-A1- 2002 010 486
- US-A1- 2021 038 238

## Beschreibung

Die Erfindung betrifft eine Haltevorrichtung für eine Lithotripsievorrichtung zum Zertrümmern von Körpersteinen, wobei die Haltevorrichtung ein Gehäuse zum Aufnehmen von Baugruppen und/oder Bauteilen aufweist, und das Gehäuse ein distales Ende und ein proximales Ende aufweist und an dem distalen Ende eine Sonotrode verbindbar ist, wobei in dem Gehäuse ein Beschleunigungsrohr als Baugruppe mit einer Längsmittelachse, einem Hohlraum, einem proximalen Ende, einem distalen Ende und mit einem bewegbaren Projektil innerhalb des Hohlraums zur Stoßanregung der Sonotrode, ein proximalseitiges Anschlagselement am proximalen Ende und ein distalseitiges Anschlagselement am distalen Ende des Beschleunigungsrohres angeordnet sind, und der Haltevorrichtung eine Krafterzeugungseinrichtung zum Erzeugen einer Kraft zum Hin- und/oder Zurückbewegen des Projektils zwischen dem proximalseitigen Anschlagselement und dem distalseitigen Anschlagselement zuordenbar ist, und in dem Gehäuse eine Schwingungsanregungseinrichtung als Baugruppe zur Schwingungsanregung der Sonotrode und eine Schwingungsdämpfungseinrichtung angeordnet sind. Des Weiteren betrifft die Erfindung eine Lithotripsievorrichtung, insbesondere intrakorporale Lithotripsievorrichtung, zum Zertrümmern von Körpersteinen.

Die Lithotripsie ist ein bekanntes Verfahren zum Zertrümmern von Körpersteinen, welche sich z.B. durch Kondensation und/oder Auskristallisation von Salzen und Eiweißen als sogenanntes Konkrement in Körperorganen, wie beispielsweise in der Blase oder Niere, bilden. Wenn die Körpersteine zu groß für einen natürlichen Abgang sind und Beschwerden verursachen, müssen diese mit einem Lithotripter zerkleinert werden, sodass die zerkleinerten Steine durch natürliche Ausscheidung und/oder mittels einer Saug-Spül-Pumpe entfernt werden können. Die zu zertrümmernden Körpersteine sind häufig inhomogen mit unterschiedlichen Bestandteilen und/oder Festigkeiten aufgebaut.

Um die Steinzertrümmerungsleistung zu verbessern, werden vor allem in der intrakorporalen Lithotripsie Kombinationssysteme eingesetzt, welche zwei verschiedene Anregungs- und/oder Schwingungsquellen kombiniert verwenden. Dazu wird häufig zusätzlich zur konstanten Ultraschallenergie eine intermittierende, ballistische Schockwellenenergie zugeführt. Dies kann beispielsweise mittels eines ballistischen Antriebs mit Elektromagneten erfolgen, bei dem ein Prallkörper mittels der Elektromagneten beschleunigt wird und auf ein Horn und/oder den Sonotrodenkopf aufschlägt. Bei pneumatischen Lithotriptern wird stattdessen das Projektil innerhalb eines Beschleunigungsrohres durch Zufuhr von Druckluft beschleunigt und die kinetische Energie des Projektils wird über einen elastischen Stoß auf das proximale Ende der Sonotrode und weiter auf deren distales Ende zum Fragmentieren eines Körpersteins übertragen.

Bei ballistischen und/oder pneumatischen Lithotripsievorrichtungen vibriert der Handgriff und die Sonotrode aufgrund der Projektilbeschleunigung vor allem stark in Längsrichtung. Bei Beschleunigung des Projektils in distaler Richtung wirkt der Druck auf das Projektil gleichzeitig auch auf das Gehäuse der Lithotripsievorrichtung, wodurch sich das Gehäuse in entgegengesetzter Richtung zurückzieht und sich die Sonotrodenspitze vom Körperstein beabstandet. Dadurch ist die Sonotrodenspitze nicht mehr optimal auf den Körperstein gerichtet und/oder verschiebt sich aufgrund fehlender Haftreibung seitlich. Bei der entgegengesetzten Rückbewegung des Projektils in proximaler Richtung bewegt sich das Gehäuse gegenläufig und damit die Sonotrodenspitze in distaler Richtung, wodurch die Gefahr besteht, dass der Körperstein von der Sonotrodenspitze weggedrückt wird, im Körpergewebe verloren geht und/oder dort Schäden anrichtet.

Zusätzlich werden in kombinierten Lithotripsievorrichtungen durch die Ultraschallanregung Quermomente auf die Sonotrode und entsprechende Vibrationen auf das Gehäuse ausgeübt. Somit wird durch diese unterschiedlich ausgerichteten Vibrationen eine genaue Ausrichtung der Sonotrodenspitze auf den Körperstein durch den Anwender erschwert und der Anwender muss wiederholt die Sonotrodenspitze während der Anwendung neu ausrichten. Zudem empfindet der Anwender unangenehme und/oder sogar schmerzhafte Vibrationen am Gehäuse und/oder Handgriff des handgehaltenen Instrumentes, welche die Handhabung und Bedienung stören.

Verschiedene Ansätze zur Verminderung von Vibrationen bei Lithotriptern, wie beispielsweise die schwingungstechnische Entkopplung von dem Gehäuse von den in dem Gehäuse aufgenommenen Baugruppen, führen häufig dazu, dass das Handstück sehr schwer wird und von dem Anwender schlecht handhabbar ist. Beispielsweise wird durch eine schwingende Lagerung in einem zweiten, umgebenden Gehäuse um das Handstück zur Entkopplung der Vibrationen von der Hand des Anwenders direkt der Durchmesser und das Gewicht erhöht, welches der Haptik und Ergonomie bei der Handhabung abträglich ist. Zudem lassen sich die Vibrationen ausgelöst durch die Beschleunigung des Projektils in distaler Richtung und dem distalen Aufprall des Projektils auch aufgrund des begrenzten Bauraumes im distalen Endabschnitt des Handstückes nur schwer entkoppeln und ein schneller und/oder harter distalseitiger Aufprall des Projektils ist insbesondere im Hinblick auf eine schnelle Zertrümmerung insbesondere von harten Steinen erwünscht.

Bei der Schwingungsanregung mittels Ultraschall ist es zudem bekannt, in Ultraschallwandlern einen Ultraschall-Schwingungskompensator auf der Gegenseite des Horns und somit am schwingenden proximalen Ende des Ultraschallkonverters anzuordnen, welcher als mechanisches Befestigungselement zwischen einem ruhenden Gehäuse der Lithotripsievorrichtung und dem schwingenden, proximalen Ende des Ultraschallkonverters dient. Bei gezielter Auslegung dieses Ultraschall-Schwingungskompensators reduziert dieser die Ultraschallschwingungen über seine Länge auf ein Minimum oder Null, ohne dass dabei der Ultraschallkonverter merklich in seiner Resonanzfrequenz verstimmt wird. Die Abmessungen eines solchen Ultraschall-Schwingungskompensators sind jedoch nicht beliebig ausbildbar, da ansonsten eine unerwünschte Verstimmung des Ultraschallkonverters erfolgt, unerwünschte Querschwingungen angeregt werden und/oder unangenehme Geräusche auftreten können. Zudem kann die Gehäuselänge in proximaler Richtung nicht frei gestaltet werden.

Aus der US 2002/0010486 A1 ist ein intrakorporaler Lithotripter bekannt. Er umfasst sowohl eine von einem elektrisch angesteuerten Piezo-Ultraschallwandler angeregte hohle Metallsonde als auch eine von einem reversibel angetriebenen Schlagteil angeregte Stoßsonde. Während der Operation kann zwischen den beiden Steinzertrümmerungsmöglichkeiten umgeschaltet werden.

Die Offenlegungsschrift DE 10 2022 109 138 A1 lehrt eine Lithotripsievorrichtung, deren Sonotrode von einem in einem Führungsrohr hin- und herbeweglichen Projektil angeregt wird. Das Projektil wird mittels einer im Führungsrohr befindlichen Steuerhülse bei kontinuierlichem Zu- und/oder Abführen eines Druckmediums selbstanregend in andauernder Bewegung gehalten.

Die Patentanmeldung US 2021/0038238 A1 betrifft eine Lithotripsievorrichtung, deren Sonde von einer ersten Antriebseinrichtung periodisch und von einer zweiten Antriebseinrichtung gepulst ausgelenkt wird.

Das Patent US 5,397,293 A offenbart eine Ultraschall-Angioplastie-Vorrichtung mit einem Ultraschallgenerator und einem ummantelten Katheter-Draht. Die Ummantelung dämpft nur die Querschwingungen des Katheter-Drahts, nicht aber dessen Aaxialbewegung.

Aufgabe der Erfindung ist es, den Stand der Technik zu verbessern.

Gelöst wird die Aufgabe durch eine Haltevorrichtung für eine Lithotripsievorrichtung zum Zertrümmern von Körpersteinen, wobei die Haltevorrichtung ein Gehäuse zum Aufnehmen von Baugruppen und/oder Bauteilen aufweist, und das Gehäuse ein distales Ende und ein proximales Ende aufweist und an dem distalen Ende eine Sonotrode verbindbar ist, wobei in dem Gehäuse ein Beschleunigungsrohr als Baugruppe mit einer Längsmittelachse, einem Hohlraum, einem proximalen Ende, einem distalen Ende und mit einem bewegbaren Projektil innerhalb des Hohlraums zur Stoßanregung der Sonotrode, ein proximalseitiges Anschlagselement am proximalen Ende und ein distalseitiges Anschlagselement am distalen Ende des Beschleunigungsrohrs angeordnet sind, und der Haltevorrichtung eine Krafterzeugungseinrichtung zum Erzeugen einer Kraft zum Hin- und/oder Zurückbewegen des Projektils zwischen dem proximalseitigen Anschlagselement und dem distalseitigen Anschlagselement zuordenbar ist, und in dem Gehäuse eine Schwingungsanregungseinrichtung als Baugruppe zur Schwingungsanregung der Sonotrode und eine Schwingungsdämpfungseinrichtung angeordnet sind, wobei die Schwingungsdämpfungseinrichtung mindestens eine Masse und mindestens zwei Federelemente mit jeweils zwei Enden aufweist, wobei die mindestens zwei Federelemente mit jeweils ihrem einen Ende die Masse und mindestens ein Federelement mit seinem zweiten Ende eine Innenoberfläche des Gehäuses kontaktiert.

Somit wird ein Handstück für eine kombinierte Lithotripsievorrichtung mit einer Schlaganregung und einer Schwingungsanregung der Sonotrode bereitgestellt, bei welchem mittels der Schwingungsdämpfungseinrichtung die aufgrund der Schlaganregung und der Schwingungsanregung induzierten Vibrationen deutlich reduziert sind. Dadurch liegt das Handstück ruhiger in der Hand des Anwenders und ist besser handhabbar, ohne dass die Impulsübertragung auf die verbindbare oder verbundene Sonotrode eingeschränkt ist. Mittels der Schwingungsdämpfungseinrichtung werden gleichzeitig unerwünschte angeregte Schwingungen erzeugt von der Schwingungsanregungseinrichtung auf das Beschleunigungsrohr oder umgekehrt erzeugt von der Krafterzeugungseinrichtung durch distalseitiges Anschlagen des Projektils auf die Schwingungsanregungseinrichtung verhindert oder zumindest vermindert, wodurch die Schwingungsanregungseinrichtung und der ballistische Antrieb mittels der Krafterzeugungseinrichtung unabhängig voneinander einstellbar und betreibbar sind.

Es ist besonders vorteilhaft, dass die Schwingungsdämpfungseinrichtung innerhalb des üblicherweise vorhandenen Bauraums in dem Gehäuse, beispielsweise proximalseitig der Schwingungsanregungseinrichtung, platzsparend integrierbar ist, sodass der Bauraum und das Gewicht des Handstückes nicht oder nur geringfügig erhöht ist. Neben der kompakten Anordnung der Schwingungsdämpfungseinrichtung innerhalb des vorhandenen Gehäuses werden zudem die Anforderungen an Sicherheit und Reinigbarkeit des Handstückes erfüllt.

Durch die Ausgestaltung der Schwingungsdämpfungseinrichtung mit mindestens einer Masse, welche als Trägheitsmasse und/oder Tilgermasse wirkt, und mit mindestens zwei Federelementen, welche als Schwingungsdämpfer wirken, ist sowohl die Intensität der Vibrationen als auch deren weitere Übertragung an die Innenoberfläche des umgebenden Gehäuses deutlich gemindert und entkoppelt. Hierbei mindert die Masse als Tilgermasse vor allem den Rückstoß nach dem Aufprall des Projektils an dem distalseitigen oder dem proximalseitigen Anschlagselement ab. Dadurch wird die Handhabbarkeit der Lithotripsievorrichtung für den Anwender und die genaue räumliche Positionierung der Sonotrodenspitze auf und/oder in dem zu zertrümmernden Körperstein verbessert, da sich die Sonotrodenspitze aufgrund der Schwingungsdämpfungseinrichtung bei der Beschleunigung des Projektils in distaler Richtung nicht so stark von dem zu zertrümmernden Körperstein beabstandet oder bei umgekehrter Beschleunigungsrichtung des Projektils die Sonotrodenspitze den Körperstein nicht ungewollt wegdrückt.

Ein wesentlicher Gedanke der Erfindung beruht darauf, durch eine in dem Gehäuse der Haltevorrichtung angeordnete Schwingungsdämpfungseinrichtung mit mindestens einer Masse und mindestens zwei Federelementen, wobei mindestens ein Federelement mit seinem einen Ende eine Innenoberfläche des Gehäuses und mit seinem anderen Ende die Masse der Schwingungsdämpfungseinrichtung kontaktiert, Vibrationen erzeugt durch eine Schwingungsanregung und eine Schlaganregung in der Lithotripsievorrichtung gezielt auszulöschen, zu dämpfen und/oder von dem Gehäuse weitgehend zu entkoppeln. Hierbei ist die Schwingungsdämpfungseinrichtung gezielt auf einen bestimmten Frequenzbereich und/oder die Betriebsfrequenz der Schwingungsanregungseinrichtung abgestimmt ist, sodass die gewünschte Schwingungsanregung und Schlaganregung der Sonotrode nicht eingeschränkt ist. Zudem ist durch eine Anordnung der Schwingungsdämpfungseinrichtung proximalseitig von der Schwingungsanregungseinrichtung auch ein Rückstoß nach dem distalseitigen Anschlag des Projektils abminderbar.

Folgendes Begriffliche sei erläutert:
Bei einer "Lithotripsievorrichtung" (auch "Lithotripter" genannt) handelt es sich insbesondere um eine Vorrichtung zum Zertrümmern von Körpersteinen durch Stöße, Stoßwellen, Verformungswellen und/oder Schwingungswellen. Unter einer Lithotripsievorrichtung werden insbesondere verschiedene Bestandteile, Bau- und/oder Funktionskomponenten eines Lithotripters verstanden. Die Lithotripsievorrichtung kann einen Lithotripter vollständig oder teilweise ausbilden. Bei einer Lithotripsievorrichtung kann es sich insbesondere um eine intrakorporale oder extrakorporale Lithotripsievorrichtung handeln. Im Falle einer intrakorporalen Lithotripsievorrichtung kann diese zusätzlich eine Spül-/Saugpumpe aufweisen. Die Lithotripsievorrichtung kann als Handgerät ausgebildet sein und/oder ein Endoskop aufweisen oder in ein Endoskop eingeschoben werden. Die Lithotripsievorrichtung ist insbesondere autoklavierbar und weist beispielsweise Instrumentenstahl und/oder Kunststoff auf. Die Lithotripsievorrichtung kann weitere Komponenten, wie ein Steuer- und/oder Versorgungsgerät aufweisen oder diese sind der Lithotripsievorrichtung zugeordnet. Eine Lithotripsievorrichtung ist insbesondere eine kombinierte Lithotripsievorrichtung mit einer ballistischen und/oder pneumatischen Einheit und zuordenbaren Krafterzeugungseinrichtung und einer Schwingungsanregungseinrichtung. Mittels der ballistischen und/oder pneumatischen Einheit und der zuordenbaren Krafterzeugungseinrichtung wird mittels einer Stoßenergie beim Anschlagen eines Projektils an einem distalseitigen Anschlagselement insbesondere der Sonotrode direkt oder indirekt eine gezielt geformte Verformungswelle aufgeprägt. Die Verformungswelle bewirkt insbesondere eine translatorische Bewegung der Sonotrode, welche aufgrund der Auslenkung eine Steinzertrümmerung bewirkt. Gleichzeitig wird bei der kombinierten Lithotripsievorrichtung neben dem mechanischen Stoß die Sonotrode zusätzlich insbesondere mittels einer Schwingungsanregungseinrichtung, beispielsweise mit einem Ultraschallwandler, in eine Schwingung, insbesondere longitudinale Schwingung und/oder Querschwingung, angeregt. Somit ist die Sonotrode insbesondere als Wellenleiter für die Schwingungswellen erzeugt von der Schwingungsanregungseinrichtung und für die Verformungswellen des Projektils ausgebildet.

Unter "Körpersteinen" (auch "Konkrement" genannt) werden insbesondere alle Steine in einem menschlichen oder tierischen Körper verstanden, welche sich z.B. aus Salzen und Eiweißen durch Kristallisation und/oder Kondensation bilden. Bei Körpersteinen kann es sich beispielsweise um Gallensteine, Harnsteine, Nierensteine und/oder Speichelsteine handeln. Durch Einwirken der Sonotrode und/oder Hohlsonde auf den Körperstein entstehen insbesondere Körpersteinkerne (auch Bohrkerne genannt) und/oder Körpersteinfragmente.

Eine "Haltevorrichtung" (auch "Handstück" genannt) ist insbesondere ein Hand- und/oder Halteteil der Lithotripsievorrichtung. Bei der Haltevorrichtung kann es sich insbesondere um eine Handhabe zur manuellen und/oder automatisierten Bedienung und/oder Verbindung der Lithotripsievorrichtung handeln. Eine Haltevorrichtung kann auch an einem distalen Ende eines Roboterarms angeordnet, verbunden und/oder automatisiert geführt sein. Die Haltevorrichtung weist insbesondere ein Gehäuse auf. Die Haltevorrichtung kann auch zwei- oder mehrteilig ausgebildet sein. Beispielsweise kann die Haltevorrichtung ein separates Gehäuse für eine Pneumatikeinheit und ein separates Gehäuse für die Schwingungsanregungseinrichtung aufweisen.

Unter "distalseitig" und "distal" wird eine patientenkörpernahe und somit benutzerferne Anordnung und/oder ein entsprechendes Ende oder Abschnitt verstanden. Dementsprechend wird unter "proximalseitig" oder "proximal" eine benutzernahe und somit patientenkörperferne Anordnung oder ein entsprechendes Ende oder Abschnitt verstanden.

Eine "Sonotrode" ist insbesondere ein Bauteil, welches durch das Einwirken und/oder Einleiten von mechanischen Schwingungen selbst in Schwingung und/oder Resonanzschwingung versetzt wird. Die Sonotrode ist insbesondere als Wellenleiter für die Schwingungswellen erzeugt von der Schwingungsanregungseinrichtung und für die Verformungswellen durch Anschlag des mittels der Krafterzeugungseinrichtung beschleunigten Projektils ausgebildet. Die Sonotrode ist insbesondere mit der Schwingungsanregungseinrichtung, dem Ultraschallwandler und/oder dem Horn direkt oder indirekt verbunden. Beispielsweise ist die Sonotrode in das distalseitige Ende des Horns eingeschraubt. Die Sonotrode weist insbesondere an ihrem proximalen Ende einen Sonotrodenkopf zum Aufnehmen, Weiterleiten und/oder Fokussieren von Ultraschallwellen und an ihrem distalen Ende eine Sonotrodenspitze zum unmittelbaren und/oder mittelbaren Beaufschlagen und/oder Kontaktieren von Körpersteinen auf. Die Sonotrode ist insbesondere derart geformt, dass diese optimal die Schwingungswellen, die Ultraschallschwingung und/die Verformungswellen an ihrem distalen Ende in den Körper, die zu behandelnde Körperregion und/oder direkt auf den zu zertrümmernden Körperstein einleitet. Im Falle der Ultraschallanregung arbeitet die Sonotrode insbesondere im Ultraschallbereich mit einem Frequenzbereich von 20 kHz bis 90 kHz, bevorzugt von 20 kHz bis 34 kHz. Die Sonotrode weist insbesondere Stahl, Titan, Aluminium und/oder Carbon auf. Bei einer Sonotrode handelt es sich insbesondere um eine Sonde, welche beispielsweise stab-, röhren- und/oder schlauchförmige ausgebildet ist. Die Sonotrode kann einstückig oder mehrteilig ausgebildet sein. Die Sonotrode weist insbesondere einen Durchmesser in einem Bereich von 0,5 mm bis 4,5 mm, insbesondere von 0,8 mm bis 3,8 mm, auf.

Ein "Beschleunigungsrohr" ist insbesondere ein länglicher Hohlkörper, dessen Länge eine größere Abmessung aufweist als sein Durchmesser. Das Beschleunigungsrohr weist in seinem Inneren insbesondere einen Hohlraum auf, in dem ein Projektil sich frei in Längsrichtung bewegen kann. Des Weiteren weist das Beschleunigungsrohr insbesondere ein proximales Ende und ein distales Ende auf, welche räumlich die maximale Beschleunigungsstrecke minus der Länge des Projektils festlegen. Das Beschleunigungsrohr ist distalseitig und/oder an seinem distalen Endabschnitt insbesondere zumindest teilweise von dem Horn und einem mit dem Horn verbundenen oder zugeordneten Bolzen umgeben. Bei einer pneumatischen Krafterzeugungseinrichtung weist das Beschleunigungsrohr zumindest eine Öffnung zum Eintritt und/oder Austritt eines Druckmediums, insbesondere Druckluft, auf. Das Beschleunigungsrohr weist insbesondere ein Metall auf.

Ein "Anschlagselement" ist insbesondere ein gewollter Endpunkt der Bewegung des Projektils entlang der Beschleunigungsstrecke innerhalb des Hohlraums des Beschleunigungsrohres, an dem das beschleunigte Projektil gegen das Anschlagselement schlägt, abgebremst und/oder in die Gegenrichtung bewegt wird. Ein distalseitiges Anschlagselement ist insbesondere am und/oder im distalen Ende des Beschleunigungsrohres und/oder innerhalb des Hohlraums in einem Bereich des distalen Abschnittes des Beschleunigungsrohres angeordnet. Das distalseitige Anschlagselement übertragt insbesondere direkt oder indirekt den Stoß des Projektils auf die Sonotrode. Bei dem distalseitigen Anschlagselement kann es sich beispielsweise um eine proximalseitige Wand des Horns, ein Federelement oder um eine proximalseitige Wand eines Halters eines Federelementes handeln. Das proximalseitige Anschlagselement ist insbesondere am und/oder im proximalen Ende des Beschleunigungsrohrs oder innerhalb des Hohlraums in einem proximalen Abschnitt des Beschleunigungsrohrs angeordnet. Bei dem proximalseitigen Anschlagselement kann es sich beispielsweise um eine Wand des Gehäuses, einer Aufnahme für das Beschleunigungsrohr und/oder um ein Federelement handeln.

Ein "Projektil" ist insbesondere ein Körper, welcher innerhalb des Hohlraums des Beschleunigungsrohres frei entlang der Beschleunigungsstrecke beweglich ist. Das Projektil ist insbesondere zwischen dem proximalseitigen Anschlagselement und dem distalseitigen Anschlagselement innerhalb des dazwischen angeordneten Hohlraums des Beschleunigungsrohres hin- und zurückbewegbar. Prinzipiell kann das Projektil jegliche Form aufweisen. Beispielsweise kann das Projektil die Form eines Bolzens oder einer Kugel aufweisen. Das Projektil weist insbesondere harten Stahl und/oder schwachmagnetische Eigenschaften auf. Für die freie Beweglichkeit weist das Projektil insbesondere einen etwas geringeren Außendurchmesser als der Durchmesser des Hohlraumes des Beschleunigungsrohres auf. Beispielsweise kann das Projektil einen Außendurchmesser von 8 mm, insbesondere 6 mm, oder 4 mm aufweisen.

Das Projektil kann insbesondere entlang der Beschleunigungsstrecke stetig oder unstetig mittels der Krafterzeugungseinrichtung hin- und/oder herbewegt werden. Bevorzugt wird das Projektil intermittierend und/oder oszillierend zwischen dem proximalseitigen Anschlagselement und dem distalseitigen Anschlagselement hin und her bewegt.

Bei einer "Krafterzeugungseinrichtung" kann es sich prinzipiell um jegliche Art von Einrichtung handeln, welche eine Kraft auf das Projektil und somit eine Bewegung des Projektils bewirkt. Bei der Krafterzeugungseinrichtung kann es sich beispielsweise um eine Vorrichtung handeln, welche mittels Lasers, eines Druckmediums, beispielsweise pneumatisch mithilfe von Druckluft, mittels eines elektromagnetischen Feldes und/oder mittels einer mechanischen Vorrichtung das Projektil beschleunigt. Eine pneumatisch Krafterzeugungseinrichtung kann insbesondere mittels eines Zuführens und/oder Abführens eines Druckmediums eine lineare Bewegung des Projektils im Hohlraum des Beschleunigungsrohres bewirken. Das Druckmedium strömt insbesondere durch mindestens eine proximalseitige Öffnung des Beschleunigungsrohres in den Hohlraum des Beschleunigungsrohres und drückt und beschleunigt das Projektil in distale Richtung.

Eine "Schwingungsanregungseinrichtung" ist insbesondere jegliche Einrichtung zum Erzeugen von Schwingungen im Ultraschallbereich. Die Schwingungsanregungseinrichtung weist insbesondere einen Ultraschallwandler (auch Ultraschallkonverter genannt) auf, welcher eine zugeführte Wechselspannung mit einer bestimmten Frequenz in eine mechanische Schwingungsfrequenz umsetzt, oder die Schwingungsanregungseinrichtung ist durch den Ultraschallwandler ausgebildet. Der Ultraschallwandler ist insbesondere ein elektromechanischer Wandler unter Ausnutzung des piezoelektrischen Effektes. Durch Anlegen der von einem Ultraschallgenerator erzeugten elektrischen Wechselspannung wird eine mechanische Schwingung aufgrund eines Verformens des Ultraschallwandlers erzeugt. Der Ultraschallwandler weist insbesondere ein Piezoelement oder mehrere, bevorzugt gestapelte, Piezoelemente auf. Bevorzugt weist der Ultraschallwandler mindestens zwei Piezoelemente auf, wobei zwischen den Piezoelementen ein elektrischer Leiter, beispielsweise eine Kupferscheibe, angeordnet ist. Ein distalseitiges Piezoelement des Ultraschallwandlers liegt insbesondere direkt an einer proximalen Wand eines Hornes an. Proximalseitig des Piezoelementes oder der Piezoelemente ist insbesondere ein Gegenlager angeordnet. Zwischen dem proximalen Ende des proximalseitigen Piezoelementes und dem distalen Ende des Gegenlagers kann eine Zwischenscheibe angeordnet sein. Das Piezoelement, die Piezoelemente, die Zwischenscheibe und/oder das Gegenlager können insbesondere um einen Bolzen, insbesondere Hohlbolzen, angeordnet sein, welcher proximalseitig des Horns angeordnet ist.

Ein "Horn" ist insbesondere ein Bauteil, welches zwischen dem Ultraschallwandler und/oder einem Piezoelement und der Sonotrode angeordnet ist. Das Horn dient insbesondere dazu, die vom Ultraschallwandler erzeugten Ultraschallwellen zur Sonotrode zu übertragen, weiterzuleiten, zu fokussieren und/oder auszurichten. Dazu kann das Horn sich in einer Übertragungsrichtung verjüngen und direkt oder indirekt die Ultraschallwellen auf einen Sondenkopf übertragen. Durch eine Querschnittsverminderung des Horns in Übertragungsrichtung wird insbesondere eine Amplitudenvergrößerung erzielt. Das Horn kann auch zur Befestigung der Sonotrode verwendet werden. Gleichzeitig dient das Horn insbesondere zusammen mit einem Gegenlager und/oder einer Zwischenscheibe zur beidseitigen mechanischen Halterung des Piezoelementes oder der Piezoelemente. Entgegen der Übertragungsrichtung, insbesondere proximalseitig, schließt das Horn mit einer Wand ab. Proximalseitig dieser Wand ist insbesondere ein Bolzen angeordnet. Bei dem Bolzen handelt es sich bevorzugt um einen Hohlbolzen. Das Horn und der Bolzen können insbesondere als zwei separate Bauteile ausgebildet sein. Bevorzugt handelt es sich bei dem Horn und dem Bolzen um ein einstückiges Bauteil, wobei ein Hornabschnitt dem konventionellen Horn entspricht und entgegen der Übertragungsrichtung, insbesondere in proximaler Richtung, insbesondere gestuft in den Hohlbolzenabschnitt mit einem geringeren Querschnitt übergeht. Um den Hohlbolzenabschnitt ist mindestens ein Piezoelement mit elektrischem Kontakt und das Gegenlager und/oder zusätzlich eine dazwischen angeordnete Zwischenscheibe zwischen dem proximalseitigen Piezoelement und der distalen Seite des Gegenlagers angeordnet. Das Gegenlager ist insbesondere auf dem Hohlbolzen oder dem Hohlbolzenabschnitt aufgeschraubt und spannt dadurch zumindest ein Piezoelement und/oder die Zwischenscheibe ein. Das Gegenlager kann als Schraubenmutter ausgebildet sein. Ein proximaler Endabschnitt des Hohlbolzenabschnittes und/oder des Hohlbolzens steht insbesondere in proximaler Richtung über das proximale Ende des Gegenlagers hinaus.

Eine "Schwingungsdämpfungseinrichtung" (auch "Schwingungstilger" genannt) ist insbesondere jegliche Einrichtung, welche Vibrationen verursacht durch die Schlaganregung mittels eines Projektils und/oder die Schwingungsanregung mittels einer Schwingungsanregungseinrichtung auslöscht, dämpft und/oder zumindest teilweise vom Gehäuse entkoppelt. Eine Schwingungsdämpfungseinrichtung ist insbesondere ein schwingungsdämpfendes Bauteil oder eine Baugruppe, welche mindestens eine Masse und mindestens zwei Federelemente aufweist, wobei mindestens ein Federelement mit seinem einen Ende die Masse der Schwingungsdämpfungseinrichtung und mit seinem anderen Ende eine Innenoberfläche des Gehäuses kontaktiert. Die Masse ist insbesondere als bewegliche Trägheitsmasse ausgebildet und wirkt als Tilgermasse, welche durch die sich ausbreitenden Schwingungen aus ihrer Ruhelage ausgelenkt wird und verzögernd wirkt, während die mindestens zwei Federelemente schwingungsdämpfend ausgebildet sind. Hierbei sind insbesondere das Gewicht der Masse und die Federkonstanten der Federelemente derart ausgebildet, dass diese auf einen gewünschten, bestimmten Frequenzbereich der Lithotripsievorrichtung und/oder eine Betriebsfrequenz eines Ultraschallschwingungsanregers der Schwingungsanregungseinrichtung abgestimmt sind und diese zulassen. Die Masse bildet zusammen mit den mindestens beiden Federelementen ein Masse-Feder-System und/oder Pendel aus, dessen Eigenfrequenz auf die zu eliminierende Schwingungsfrequenz oder Schwingungsfrequenzen eingestellt ist. Bevorzugt ist die Eigenfrequenz der Schwingungsdämpfungseinrichtung auf eine zu eliminierende, unerwünschte Frequenz und/oder Resonanzfrequenz einer schwingenden Baugruppe, wie der Schwingungsanregungseinrichtung, eingestellt. Bei dieser Frequenz kann der Schwingungstilger große Schwingungsauslenkungen ausführen, wobei der Schwingungstilger der schwingenden Baugruppe Schwingungsenergie für seine eigene Schwingungsbewegung entzieht, welche aufgrund von Reibung in Wärme umgewandelt und dadurch ausgelöscht wird. Somit verhindert und/oder vermindert die Schwingungsdämpfungseinrichtung insbesondere die Ausbreitung und Übertragung von Schwingungen über Bauteile und/oder Baugruppen innerhalb des Gehäuses, welche ansonsten als Vibrationen auf das Gehäuse und/oder das Handstück übertragen werden könnten.

Ein "Federelement" ist insbesondere ein Bauteil und/oder ein Abschnitt der Schwingungsdämpfungseinrichtung, welches sich ausreichend elastisch verformen lässt. Das Federelement weist insbesondere Metall und/oder Kunststoff auf. Bei einem Federelement kann es sich insbesondere um eine konventionelle Feder, wie beispielsweise eine Schraubenfeder und somit einen in Schraubenform gewickelten Draht, handeln. Die elastische Verformung des Federelements ist insbesondere eine Biegung, Torsion, Dehnung und/oder Stauchung. Neben der Schwingungsdämpfung dient das Federelement insbesondere Zum Halten und/oder Rückstellen der der Masse in ihre Ruhelage. Dazu stützen sich bevorzugt beidseitig jeweils ein Federelement an den gegenüberliegenden Stirnseiten der Masse ab. Das Federelement wird insbesondere bei einer proximalseitigen Anordnung innerhalb des Gehäuses bei einer Beschleunigungsrichtung des Projektils in distaler Richtung gedehnt und/oder bei einer Beschleunigungsrichtung des Projektils in proximaler Richtung zusammengedrückt.

Eine "Schwingungskompensationseinrichtung" (auch "Amplituden-Kompensator" genannt) ist insbesondere ein Bauteil oder eine Baugruppe, welche mindestens eine Masse und mindestens ein Federelement aufweist. Die Schwingungskompensationseinrichtung dient insbesondere zur schwingungstechnischen Entkopplung des Beschleunigungsrohres des ballistischen und/oder pneumatischen Antriebs von der Schwingungsanregung mittels der Schwingungsanregungseinrichtung. Das Federelement ist insbesondere auf der distalen Seite und die Masse als Ruhemasse auf der proximalen Seite der Schwingungskompensationseinrichtung angeordnet. Die Schwingungskompensationseinrichtung weist insbesondere einen durchgehenden Hohlraum in ihrer Masse und ihrem Federelement auf, durch welchen das Beschleunigungsrohr durchführbar ist, sodass das Beschleunigungsrohr an seiner Außenoberfläche von der Schwingungskompensationseinrichtung umgeben ist.

Die Schwingungskompensationseinrichtung weist als weitere Bauteile insbesondere mindestens ein Verbindungselement zum Verbinden an den Bolzen und/oder das Horn des Ultraschallwandlers und mindestens ein Dichtungselement, wie beispielsweise einen O-Ring auf. Das Dichtungselement wirkt gleichzeitig als Dämpfungselement. Die Schwingungskompensationseinrichtung kann auch mehrere, beispielsweise parallel zueinander angeordnete Federelemente und/oder mehrere Massen aufweisen. Bei dem Federelement der Schwingungskompensationseinrichtung handelt es sich insbesondere um einen dünnwandigen Rohrabschnitt, welcher insbesondere als λ/4-Masse-Federlement wirkt. Die Masse und/oder die gesamte Schwingungskompensationseinrichtung weist insbesondere Aluminium und/oder Stahl auf. Bevorzugt weist der gesamte Amplituden-Kompensator Aluminium und/oder eine Aluminiumlegierung auf. Während im Betrieb das Federelement der Schwingungskompensationseinrichtung schwingt und dadurch dämpfend wirkt, bleibt die Masse aufgrund ihres deutlich größeren Gewichtes insbesondere in Ruhe und schwingt gerade nicht.

Eine "Längsmittelachse" ist insbesondere die Achse des jeweiligen Körpers oder Bauteiles, welche der Richtung seiner größten Ausdehnung und/oder Abmessung entspricht. Bei der Längsmittelachse kann es sich auch um die Symmetrieachse des jeweiligen Körpers und/oder Bauteiles handeln.

Eine "Längsrichtung" ist insbesondere die Richtung der längsten Ausdehnung eines Bauteils und/oder Körpers. Die Längsrichtung ist insbesondere die Richtung entlang der Längsmittelachse der Masse, der Sonotrode und/oder des Beschleunigungsrohrs.

In einer weiteren Ausführungsform der Haltevorrichtung weist die Schwingungsdämpfungseinrichtung ein drittes Federelement und optional weitere Federelemente auf.

Durch ein drittes Federelement und optional weitere Federelemente kann sowohl eine stärkere Entkopplung zum Gehäuse erzielt werden, indem eines der beiden Enden des dritten und/oder des jeweils weiteren Federelementes die Masse und das andere Ende die Innenoberfläche des Gehäuses kontaktiert. Ebenso kann das zweite Ende des dritten und/oder jeweils weiteren Federelementes statt der Innenoberfläche des Gehäuses auch eine Baugruppe, ein Bauteil und/oder eine Halteaufnahme innerhalb des Gehäuses kontaktieren. Durch das dritte Federelement und/oder weitere Federelemente kann die Schwingungsdämpfung und/oder -entkopplung gezielt durch Ausrichtung und Ausbildung des jeweiligen Federelementes in unterschiedliche Richtungen und/oder mit unterschiedlichen Intensitäten gedämpft und entkoppelt werden. Prinzipiell ist herauszustellen, dass die zwei, drei oder mehreren Federelemente gleichartig ausgebildet sein können, jedoch auch unterschiedliche Eigenschaften, wie unterschiedliche Längen, Federkonstanten und/oder Abmessungen, aufweisen können.

Um eine kompakte Anordnung der Schwingungsdämpfungseinrichtung und eine optimale Ausnutzung des vorhandenen Bauraums innerhalb des Gehäuses der Haltevorrichtung zu realisieren, kann eine Baugruppe und/oder können mehrere Baugruppen oder alle Baugruppen in dem Gehäuse als jeweils eine Masse der Schwingungsdämpfungseinrichtung ausgebildet sein.

Somit muss die Schwingungsdämpfungseinrichtung nicht eine separate, eigene Masse aufweisen, sondern die in dem Gehäuse bereits vorhandenen Komponenten können jeweils als Masse eingesetzt werden, welche mit mindestens einem Federelement in Kontakt steht. Vor allem in dem Fall, in dem mehrere Baugruppen oder alle Baugruppen im Inneren des Gehäuses jeweils als Masse der Schwingungsdämpfungseinrichtung ausgebildet sind, wird eine optimale Dämpfung und Entkopplung von induzierten Vibrationen in einer Vielzahl von unterschiedlichen Raumrichtungen erzielt, sodass die gesamte Außenoberfläche des Handstückes vibrationsarm ausgebildet ist.

In einer weiteren Ausführungsform der Haltevorrichtung ist die Schwingungsanregungseinrichtung oder ein Bauteil der Schwingungsanregungseinrichtung als eine Masse der Schwingungsdämpfungseinrichtung ausgebildet.

Dadurch kann eine Schwingungstilgung direkt in der Schwingungsanregungseinrichtung selbst realisiert und zum Dämpfen von unerwünschten Schwingungen eingestellt sein. Beispielsweise kann das Gegenlager eines Ultraschallwandlers als Masse ausgebildet und direkt mit einem Federelement verbunden sein, welches an seinem anderen Ende die Innenoberfläche des Gehäuses oder eine Baugruppe und/oder ein Bauteil innerhalb des Gehäuses kontaktiert.

Um eine Schwingungstilgung in Längsrichtung des Gehäuses zwischen dem proximalseitigen Anschlagselement und dem distalseitigen Anschlagselement zu erzielen, ist das Beschleunigungsrohr als eine Masse der Schwingungsdämpfungseinrichtung ausgebildet.

Somit kann eine Schwingungstilgung mittels des Beschleunigungsrohrs als Masse über die gesamte Länge des Beschleunigungsrohres erfolgen, wobei die Federelemente an definierten Positionen entlang der Längsrichtung des Beschleunigungsrohres anordenbar sind, an welchen eine gezielte Schwingungsdämpfung zu der Innenoberfläche des Gehäuses und/oder eines anderen Bauteils eingestellt werden soll.

In einer weiteren Ausführungsform der Haltevorrichtung weist das Gehäuse einen Platinenhalter auf, wobei der Platinenhalter als eine Masse der Schwingungsdämpfungseinrichtung ausgebildet ist.

Somit weist der Platinenhalter die Doppelfunktion als Trägerelement für elektronische Bauteile innerhalb der Haltevorrichtung als auch als Masse der Schwingungsdämpfungseinrichtung auf.

Um die Schwingungsdämpfungseinrichtung optimal innerhalb des vorhandenen Bauraums in dem langgestreckten Gehäuse zu integrieren, sind die beiden Federelemente auf jeweils einer Seite der Masse in Längsrichtung angeordnet und mittels einer Halteeinheit gehalten.

Dadurch kann die Schwingungsdämpfungseinrichtung als eine einstückige kompakte Baugruppe in einen vorhandenen Freiraum innerhalb des Gehäuses der Haltevorrichtung eingesetzt werden.

Eine "Halteeinheit" ist insbesondere eine Halterung zum Halten und/oder Befestigen der Schwingungsdämpfungseinrichtung. Die Halteeinheit umgibt und/oder trägt zumindest teilweise die Masse und/oder die Federelemente der Schwingungsdämpfungseinrichtung. Das jeweilige Federelement kann beispielsweise die Halteeinheit umgeben oder die Halteeinheit ist um das jeweilige Federelement angeordnet. Das Ende des Federelementes, welches gegenüber dem Ende an der Masse angeordnet ist, kann insbesondere gegen einen Bestandteil der Halteeinheit drücken, sodass die Halteeinheit ein Stützlager für das Federelement gegenüberliegend zur Masse ausbildet. Jedoch kann die Halteeinheit auch eine freie Stirnseite an dem Ende des Federelementes auf, welches die Innenoberfläche des Gehäuses und/oder eine Baugruppe kontaktiert. Somit kann es sich bei der Halteeinheit beispielsweise um ein Rohr handeln, in welchem die Schwingungsdämpfungseinrichtung angeordnet ist, wobei an den freien Rohrenden jeweils ein Ende eines Federelementes angeordnet ist, welche eine Innenoberfläche des Gehäuses und/oder eine Baugruppe kontaktiert. Ebenso kann es sich bei der Halteeinheit um einen Kolben, einen Stab oder eine Schiene handeln, welcher oder welche beispielsweise beidseitig der Masse angeordnet ist und jeweils von einem Federelement umgeben ist.

In einer weiteren Ausführungsform der Haltevorrichtung sind ein erstes Federelement proximalseitig der Schwingungsanregungseinrichtung und ein zweites Federelement distalseitig des proximalen Endes des Gehäuses angeordnet.

Dadurch wird eine optimale Dämpfung und Entkopplung von Vibrationen verursacht durch den Rückstoß des Projektils nach Anschlagen an dem distalseitigen oder proximalseitigen Anschlagselement erreicht.

Um eine verbesserte Schwingungsentkopplung zwischen der Ultraschalleinheit und dem Beschleunigungsrohr zu erreichen, kann zwischen der Schwingungsanregungseinrichtung und dem ersten Federelement eine Schwingungskompensationseinrichtung angeordnet sein.

Somit werden Vibrationen des Handstückes weiter reduziert, da mittels der Schwingungskompensationseinrichtung eine zusätzliche Kompensation von Ultraschallschwingungen erfolgt und unerwünschte angeregte Schwingungen erzeugt von der Schwingungsanregungseinrichtung auf das Beschleunigungsrohr verhindert oder zumindest vermindert werden, wodurch die Schwingungsanregungseinrichtung und der ballistische und/oder pneumatische Antrieb mittels der Krafterzeugungseinrichtung unabhängig voneinander einstellbar und betreibbar sind. Folglich werden in proximaler Richtung zunächst unerwünschte Ultraschallschwingungen und/oder Quermomente von der Schwingungskompensationseinrichtung kompensiert und anschließend von der Schwingungsdämpfungseinrichtung weiter getilgt und gedämpft. Des Weiteren können sich das Horn, der Bolzen und/oder der Ultraschallwandler sowie die Schwingungskompensationseinrichtung innerhalb des Gehäuses bewegen und werden mittels der Schwingungsdämpfungseinrichtung von der Innenoberfläche des Gehäuses entkoppelt.

In einer weiteren Ausführungsform ist die Masse konzentrisch um das Beschleunigungsrohr angeordnet, wobei die Federelemente jeweils mit ihrem einen Ende eine Außenoberfläche der Masse und mit ihrem anderen Ende die Innenoberfläche des Gehäuses kontaktieren.

Durch die konzentrische Anordnung der Masse als Tilgermasse um das Beschleunigungsrohr kann diese Masse aufgrund der zwischen der Masse und dem Gehäuse angeordneten Federelemente in der Art eines Pendels dreidimensional frei um das Beschleunigungsrohr schwingen. Dadurch dass die Masse konzentrisch das Beschleunigungsrohr umgibt, werden optimal Schwingungen ausgehend von dem Beschleunigungsrohr oder wirkend auf das Beschleunigungsrohr gedämpft, ohne dass ein mechanischer Kontakt zwischen dem Beschleunigungsrohr und der Masse besteht. Es ist besonders vorteilhaft, wenn hierbei die Federelemente zwischen der Außenoberfläche der Masse und der Innenoberfläche des Gehäuses gleichmäßig über den Querschnitt der konzentrisch angeordneten Masse verteilt sind. Bei der Masse kann es sich hierbei beispielsweise um einen Platinenhalter handeln, welcher insbesondere im proximalen Bereich des Beschleunigungsrohres konzentrisch um das Beschleunigungsrohr angeordnet ist. Neben einem radial kompakten Design innerhalb des Gehäuses des Handstückes wird zudem eine radial gleichmäßig ausgerichtete Schwingungstilgung und -dämpfung erzielt.

Um die Schwingungen und somit die Vibrationen weiter zu dämpfen, weist das jeweilige Federelement und/oder die Halteeinheit eine Stoßdämpfereinheit auf.

Somit klingen die Schwingungen, welche auf die Schwingungsdämpfungseinrichtung und/oder die bewegliche Masse wirken, aufgrund der Stoßdämpfereinheit schneller ab und/oder werden stärker vermindert.

Eine "Stoßdämpfereinheit" ist insbesondere ein Bauteil, welches die Schwingungen der Schwingungsdämpfungseinrichtung und/oder der beweglichen Masse der Schwingungsdämpfungseinrichtung schneller abklingen lässt. Die Stoßdämpfereinheit wandelt insbesondere die Schwingungsenergie in Wärme um, wodurch die Schwingungen deutlich gedämpft werden und schneller abklingen. Bei einer Stoßdämpfereinheit kann es sich um einen hydraulischen Dämpfer mit einem Hydraulikfluid oder einen Reibungsdämpfer handeln.

In einer weiteren Ausführungsform weist die Haltevorrichtung distalseitig ein Horn und proximalseitig vom Horn einen Bolzen auf, wobei das Horn und der Bolzen einen distalen Abschnitt des Beschleunigungsrohrs umgeben, proximalseitig von dem Horn ein Gegenlager an dem Bolzen angeordnet ist und zwischen dem Gegenlager und dem Horn mindestens ein Piezoelement als Schwingungsanreger angeordnet und mechanisch gekoppelt ist, wobei das Horn das distalseitige Anschlagselement aufweist und/oder das Horn mit dem distalseitigen Anschlagselement und/oder der Sonotrode verbindbar ist und das mindestens eine Piezoelement elektrisch mit einem zuordenbaren Ultraschallgenerator verbindbar ist, wobei die Schwingungsdämpfungseinrichtung proximalseitig an und/oder von dem Horn, dem Bolzen und/oder dem Gegenlager angeordnet ist.

Durch die proximalseitige Anordnung der Schwingungsdämpfungseinrichtung direkt vom Ultraschallwandler und/oder eine Komponente des Ultraschallwandlers können gezielt unerwünschte Schwingungen in proximaler Richtung und/oder Querrichtung angeregt durch den Ultraschallwandler getilgt und gedämpft werden, sodass eine weitere Übertragung in proximaler Richtung innerhalb des Gehäuses verhindert ist.

In einem weiteren Aspekt der Erfindung wird die Aufgabe gelöst durch eine Lithotripsievorrichtung, insbesondere intrakorporale Lithotripsievorrichtung, zum Zertrümmern von Körpersteinen, wobei die Lithotripsievorrichtung eine Sonotrode und eine Haltevorrichtung aufweist, und die Haltevorrichtung eine zuvor beschriebene Haltevorrichtung ist.

Somit wird eine Lithotripsievorrichtung mit einem Handstück bereitgestellt, bei der unerwünschte Vibrationen des Handstückes aufgrund der Schwingungsdämpfungseinrichtung in dem Handstück weitgehend verhindert sind, eine effiziente Ausnutzung des Bauraumes innerhalb des Gehäuses und die gezielte Handhabung des Handstückes und somit der Lithotripsievorrichtung durch den Anwender ermöglicht sind, ohne dass die erwünschte Impulsübertragung mittels Schwingungsanregung und Schlaganregung auf die Sonotrode zum Zertrümmern von Körpersteinen eingeschränkt ist. Die Zeichnungen, die Beschreibung und die Ansprüche enthalten zahlreiche Merkmale in Kombination. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Im Weiteren wird die Erfindung anhand von Ausführungsbeispielen erläutert. Es zeigen
- Fig. 1: eine schematische dreidimensionale Darstellung einer Lithotripsievorrichtung mit einem Handstück, einem Horn und einer Sonotrode,
- Fig. 2: eine schematische Darstellung des Handstückes aus Figur 1 mit dem Horn, einem Amplituden-Kompensator, einem Schwingungstilger und einem Beschleunigungsrohr im Vollschnitt,
- Fig. 3: eine stark schematische Darstellung des Schwingungstilgers aus Figur 2, und
- Fig. 4: eine stark schematische Darstellung eines Handstückes und einer Alternative eines Schwingungstilgers und mit einer Masse konzentrisch angeordnet um ein Beschleunigungsrohr und drei Federelementen verbunden mit einem Gehäuse des Handstückes.

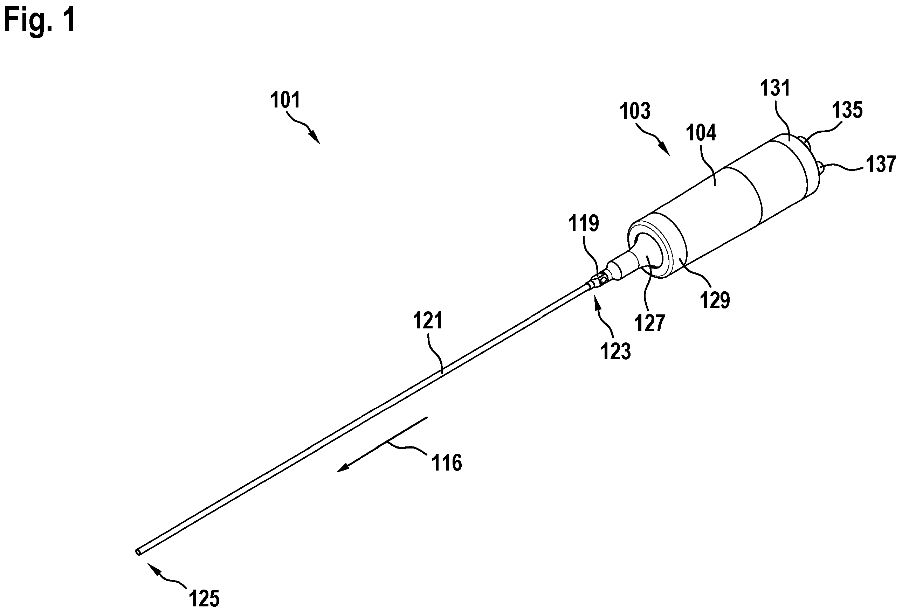

Eine Lithotripsievorrichtung 101 weist ein Handstück 103 mit einem Gehäuse 104 auf. An seinem proximalen Ende ist das Gehäuse 104 mit einem Deckel 131 abgeschlossen. An dem Deckel 131 sind proximalseitig ein elektrischer Anschluss 135 und ein Verbindungsstutzen 137 zum Zuführen von Druckluft angeordnet. Distalseitig weist das Gehäuse 104 eine Hülse 129 auf, welche ein Horn 127 umgibt. Eine Sonotrode 121 ist an ihrem proximalen Ende 123 mittels ihres Sonotrodenkopfes 119 in dem Horn 127 eingeschraubt. Ein dem proximalen Ende 123 gegenüberliegendes distales Ende 125 der Sonotrode 121 dient zum Zertrümmern von Körpersteinen (Figur 1).

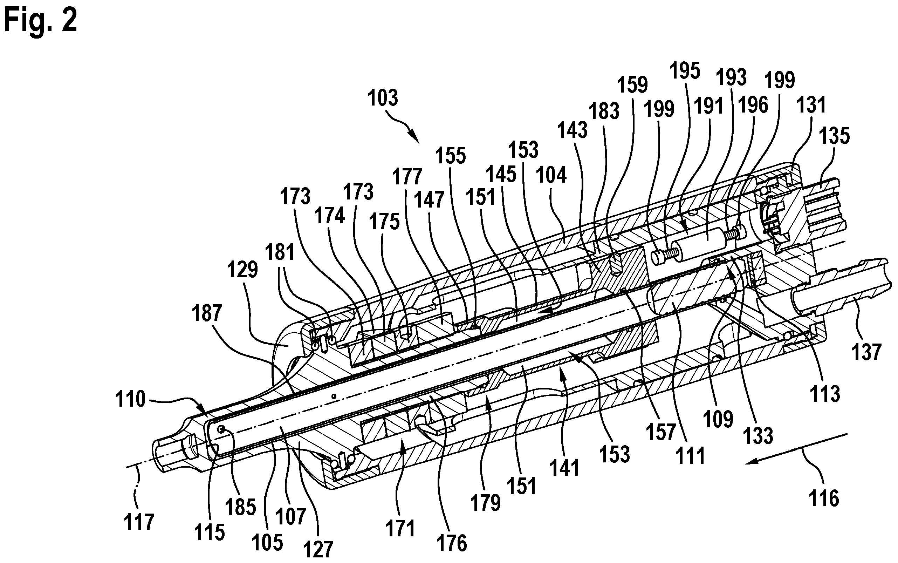

Das Horn 127 weist in einer distalen Richtung 116 einen sich verjüngenden Abschnitt auf. Proximalseitig dieses sich verjüngenden Abschnittes geht das Horn 127 einstückig in einen Hohlbolzen 176 über. Das Horn 127 ist mittels zweier O-Ringe 181 an seinem größten Querschnitt im Gehäuse 104 gelagert. Innenliegend in dem hohl ausgebildeten Horn 127 und dem sich anschließenden Hohlbolzen 176 ist ein Beschleunigungsrohr 105 angeordnet, welches sich von seinem distalen Ende 110 bis zu seinem proximalen Ende 109 entlang einer Längsmittelachse 117 erstreckt (siehe Figur 2). Das Beschleunigungsrohr 105 weist innenliegend einen Hohlraum 107 auf, in dem ein Projektil 111 beweglich angeordnet ist. Das proximale Ende 109 des Beschleunigungsrohrs 105 ist in einer Rohraufnahme 133 innerhalb des Gehäuses 104 gehalten. Der Hohlraum 107 des Beschleunigungsrohres 105 ist fluidtechnisch mit dem Verbindungsstutzen 137 verbunden. Entlang der Längsmittelachse 117 ist das Projektil 111 in dem Hohlraum 107 des Beschleunigungsrohres 105 zwischen einem proximalseitigen Anschlagselement 113 und einem distalseitigen Anschlagselement 115 beweglich.

Das distalseitige Anschlagselement 115 wird durch eine proximalseitige Wand des Horns 127 ausgebildet.

Distalseitig ist um den Hohlbolzen 176 ein Ultraschallwandler 171 angeordnet. Der Ultraschallwandler 171 weist zwei Piezoelemente 173 mit einem dazwischen angeordneten elektrischen Leiter und einem elektrischen Kontakt 174 auf. Die Piezoelemente 173 sind zwischen dem Horn 127 und einer Zwischenscheibe 175 mittels eines proximalseitigen Gegenlager 177 eingespannt, wobei die Zwischenscheibe 175 und das Gegenlager 177 ebenfalls den Hohlbolzen 176 umgeben. Am proximalen Ende 179 des Ultraschallwandlers 171 und im mittleren Bereich des Gehäuses 104 ist ein Amplituden-Kompensator 141 um das Beschleunigungsrohr 105 angeordnet. Der Amplituden-Kompensator 141 ist einstückig aus Aluminium gefertigt und weist proximalseitig ein Masseteil 143 und distalseitig einen Feder-Rohrabschnitt 145 auf. Der Feder-Rohrabschnitt 145 weist an seinem distalen Ende einen Verbindungsabschnitt 147 auf. Der Verbindungsabschnitt 147 ist auf das proximale Ende des Hohlbolzens 176 aufgeschraubt und mittels eines innenliegenden distalen O-Ringes 155 abgedichtet. Der Amplituden-Kompensator 141 weist innenliegend einen Hohlraum auf, durch den das Beschleunigungsrohr 105 geführt ist. Zusätzlich weist der Amplituden-Kompensator 141 in seiner Innenwand um den Hohlraum eine Aussparung 151 auf, welche in den Feder-Rohrabschnitt 145 und einen distalen Abschnitt des Masseteils 143 eingebracht ist, sodass der Amplituden-Kompensator 141 rundumlaufend um das Beschleunigungsrohr 105 ein Druckluftreservoir 153 aufweist (Figur 2).

Das Masseteil 143 ist mit einem proximalen O-Ring 157 an dem Beschleunigungsrohr 105 abgedichtet. Dadurch, dass der Amplituden-Kompensator 141 lediglich mit dem proximalen O-Ring 157 proximalseitig an dem Beschleunigungsrohr 105 abgedichtet ist, kann Druckluft in das durch die Aussparung 151 ausgebildete Druckluftreservoir 153 distalseitig durch einen Druckluftkanal 187 zwischen der Außenoberfläche des Beschleunigungsrohrs 105 und der Innenoberfläche des distalen Abschnittes des Amplituden-Kompensators 141, des Hohlbolzens 176 und des Horns 127 in distaler Richtung 116 aus dem Druckluftreservoir 153 austreten und durch eine Öffnung 185 am distalen Ende 110 des Beschleunigungsrohres 105 und/oder durch die offene Stirnseite am distalen Ende 110 des Beschleunigungsrohrs 105 in den Hohlraum 107 einströmen. Ebenso kann umgekehrt Druckluft aus dem Hohlraum 107 bei einer Beschleunigung des Projektils 111 in distaler Richtung 116 durch die Öffnung 185 und die offenen Stirnseite am distalen Ende 110 des Beschleunigungsrohrs 105 in den Druckluftkanal 187 als Zwischenraum zwischen der Außenoberfläche des Beschleunigungsrohrs 105 und der Innenoberfläche des Horns 127, des Hohlbolzens 176 des distalen Abschnittes des Amplituden-Kompensators 141 entgegen der distalen Richtung 116 in das Druckluftreservoir 153 gedrückt und dort gesammelt werden. Hierbei dichtet der distale O-Ring 155 zwischen dem Verbindungsabschnitt 147 des Feder-Rohrabschnittes 145 und dem proximalen Ende des Hohlbolzens 176 den Druckluftkanal 187 zum Innenraum des Gehäuses 104 ab.

Ein Platinenhalter 183 umgibt das Beschleunigungsrohr 105 von seinem proximalen Ende 109 in distaler Richtung 116 bis einschließlich des Amplituden-Kompensators 141 und des Gegenhalters 177. Das Masseteil 143 des Amplituden-Kompensators 141 ist an seiner Mantelfläche mittels drei radial gleichmäßig beabstandeter Kunststoff-Stifte 159 punktuell in Riffeln an der Innenoberfläche des Platinenhalters 183 kraft- und formschlüssig befestigt. Der Platinenhalter 183 steht wiederum radial umlaufend mit der Innenseite des Gehäuses 104 in Kontakt, sodass der Amplituden-Kompensator 141 indirekt über den Platinenhalter 183 mit dem Gehäuse 104 in radialer Richtung verbunden ist. Dadurch ist das proximale Ende des Masseteils 143 gerade frei von einer Verbindung zu dem Gehäuse 104 und dem Deckel 131 in proximaler Richtung.

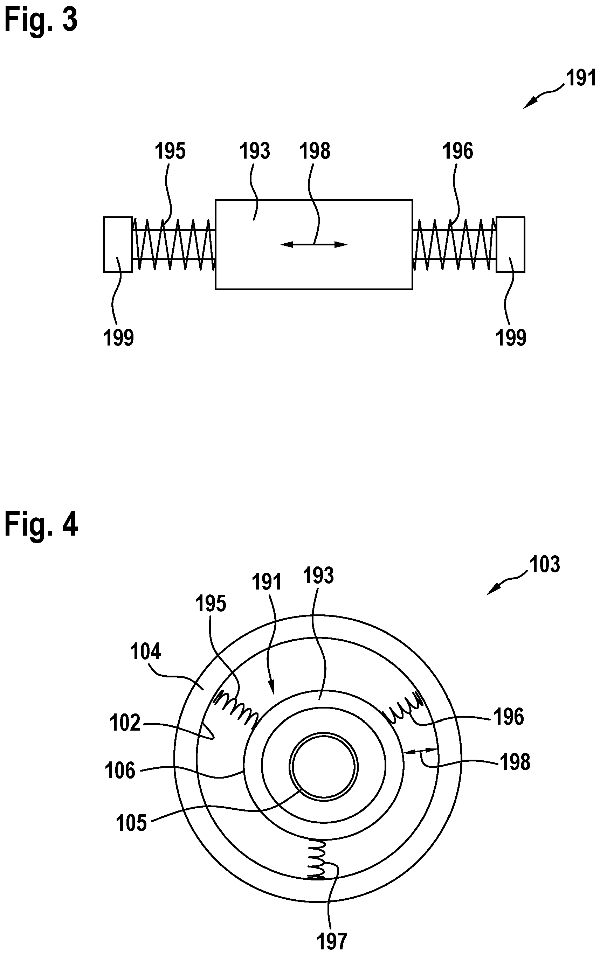

Zwischen der proximalseitigen Wand des Masseteils 143 des Amplituden-Kompensators 141 und der distalseitigen Wand der Rohraufnahme 133 ist ein Schwingungstilger 191 angeordnet. Der Schwingungstilger 191 weist eine Masse 193 als Tilgermasse und an seinen gegenüberliegenden Stirnseiten eine erste Druckfeder 195 und eine zweite Druckfeder 196 auf. Die erste Druckfeder 195 und die zweite Druckfeder 196 sind mittels einer Halterung 199 gehalten, wobei die erste Druckfeder 195 und die zweite Druckfeder 196 jeweils um einen Kolben der Halterung 199 angeordnet sind (siehe Figuren 2 und 3). Aufgrund der beidseitig angeordneten ersten Druckfeder 195 und der zweiten Druckfeder 196 ist die Masse 193 in einer gegenläufigen Dämpfungsrichtung 198 beidseitig beweglich gelagert.

Mit der kombinierten Lithotripsievorrichtung 101 mit einer Schwingungsanregung der Sonotrode 121 mittels des Ultraschallwandlers 171 und einem pneumatischen Antrieb zur Stoßanregung der Sonotrode 121 mittels des Projektils 111 werden folgende Arbeitsgänge durchgeführt.

Mittels eines nicht in den Figuren gezeigten Ultraschallgenerators wird der Ultraschallwandler 171 mit einer Spannung an dem elektrischen Kontakt 174 beaufschlagt, wodurch eine Verformung der Piezoelemente 173 innerhalb des Ultraschallwandlers 171 erfolgt und dadurch eine Ultraschallschwingung indiziert wird. Die erzeugte Ultraschallschwingung wird aufgrund des konischen Abschnittes des Horns 127 in die Sonotrode 121 eingeleitet, wodurch die Sonotrode 121 zu einer Schwingungswelle in einer longitudinalen Schwingung als auch in Querrichtung angeregt wird.

Gleichzeitig wird mittels einer nicht gezeigten Krafterzeugungseinrichtung Druckluft durch den Verbindungsstutzen 137 in den Hohlraum 107 am proximalen Ende 109 des Beschleunigungsrohrs 105 gedrückt, wodurch sich das Projektil 111 vom proximalen Ende 109 als Ausgangszustand (siehe Figur 2) in distaler Richtung 116 durch den Hohlraum 107 entlang der Längsmittelachse 117 vom proximalseitigen Anschlagselement 113 zum distalseitigen Anschlagselement 115 bewegt. Durch Aufschlagen des Projektils 111 auf das distalseitige Anschlagselement 115 wird über das distale Ende des Horns 127 und den Sonotrodenkopf 119 der Schlag der Projektils 111 auf die Sonotrode 121 übertragen wird. Durch die Beschleunigung des Projektils 111 in distaler Richtung 116 wird die Luft im distalen Abschnitt des Hohlraumes 107 innerhalb des Beschleunigungsrohres 105 komprimiert und entweicht durch die Öffnung 185 und den Druckluftkanal 187 zwischen der Außenoberfläche des Beschleunigungsrohrs 105 und der Innenoberfläche des Hornes 127, des Hohlbolzens 176 und des distalen Abschnittes des Amplituden-Kompensators 141 entgegen der distalen Richtung 116 in das Druckluftreservoir 153 des Amplituden-Kompensators 141, wobei die Druckluft in dem Druckluftreservoir 153 komprimiert wird. Durch Aufprall des Projektils 111 gegen das distalseitige Anschlagselement 115 wird das Projektil 111 repulsiert und durch gleichzeitiges Schließen der zuströmenden Druckluft durch den Verbindungsstutzen 137 strömt nun die in dem Druckluftreservoir 153 komprimierte Druckluft in distaler Richtung 116 durch den Druckluftkanal 187, die Öffnung 185 und die offene distale Stirnseite des Beschleunigungsrohrs 105 in den Hohlraum 107 und drückt das Projektil 111 wieder zum proximalen Ende 109 des Beschleunigungsrohres 105 zurück, bis der Ausgangszustand (Figur 2) wieder erreicht ist. Diese Schlaganregung der Sonotrode 121 durch Auftreffen des Projektils 111 auf das distalseitige Anschlagselement 115 wird regelmäßig wiederholt.

Die mittels des Ultraschallwandler 171 erzeugte Ultraschallschwingungen weisen eine Frequenz von circa 27 kHz auf, auf welche der Amplituden-Kompensator 141 exakt abgestimmt ist. Dadurch, dass der Amplituden-Kompensator 141 eine λ/4-Geometrie aufweist, welche der Resonanzfrequenz des Ultraschallwandlers 171 entspricht, wird der Ultraschallwandler 171 durch den Amplituden-Kompensator 141 nicht verstimmt. Aufgrund der λ/4-Geometrie des Amplituden-Kompensators 141 trifft die mittels des Ultraschallwandlers 171 erzeugte Schwingungswelle mit ihrem Amplitudenmaximum bei einer Viertelwellenlänge auf den Feder-Rohrabschnitt 145, welcher aufgrund seiner elastischen Eigenschaften schwingt und diese Amplitude aufnimmt und dämpft, sodass das Masseteil 143 als Ruhemasse sich aufgrund der geringen Rest-Ultraschallamplitude, wenn überhaupt, nur vernachlässigbar gering bewegt. Hierbei wirken die radial umlaufend angeordneten Kunststoff-Stifte 159 zur punktuellen Lagerung und der proximale O-Ring 157 zusätzlich dämpfend, sodass ein Abrieb, anderweitige Beschädigung und eine Erwärmung im Masseteil 143 vernachlässigbar sind. Durch die punktuelle Lagerung mittels der Kunststoff-Stifte 159, über welche etwaig vorhandene Quermomente radial nach außen abgegeben werden, wird zudem ein metallisches Scheppern an dem Platinenhalter 183 verhindert.

Dadurch, dass der Amplituden-Kompensator 141 mittels der Kunststoff-Stifte 159 an seiner Mantelfläche radial nach außen an dem Platinenhalter 183 gelagert ist und das proximale Ende des Masseteils 143 frei in proximaler Richtung und gerade nicht mit dem Gehäuse 104 und dem Deckel 131 verbunden ist, wird das Beschleunigungsrohr 105 optimal schwingungstechnisch mittels des Amplituden-Kompensator 141 von dem Ultraschallwandler 171 entkoppelt, sodass der pneumatische Antrieb des Projektils 111 im Beschleunigungsrohr 105 unabhängig von der erzeugten Ultraschallschwingung mittels des Ultraschallwandlers 171 betreibbar ist und beide Antriebe unabhängig voneinander einstellbar sind.

Etwaige Restschwingungen, welche trotz des Amplituden-Kompensators 141 erzeugt von dem Ultraschallwandler 171 proximalseitig des Amplituden-Kompensators 141 auftreten und unerwünschte Vibrationen des Gehäuses 104 bewirken können, werden mittels des Schwingungstilgers 191 aufgrund dessen Masse 193 getilgt und mittels der ersten Druckfeder 195 und der zweiten Druckfeder 196 gedämpft. Hierbei ist der Schwingungstilger 191 auf die zu eliminierende Frequenz der Restschwingungen abgestimmt, während die erwünschte Frequenz von 27 kHz des Ultraschallwandler 171 nicht beeinträchtigt wird. Angeregt durch die Restschwingungen führt die bewegliche Masse 193 eine große Auslenkbewegung wechselseitig in der Dämpfungsrichtung 198 aus, wobei für diese Auslenkung Schwingungsenergie entzogen wird, welche mittels der ersten Druckfeder 195 und der zweite Druckfeder 196 aufgrund von Reibung in Wärme umgesetzt wird, sodass die Restschwingungen gemindert werden.

Bei der oben beschriebenen Beschleunigung des Projektils 111 in distaler Richtung 119 wirkt der Druck auf das Projektil 111 gleichzeitig auch auf das Gehäuse 104 des Handgriffes 103, wodurch sich das Gehäuse 104 in entgegengesetzte Richtung zurückzieht und die erste Druckfeder 195 und die zweite Druckfeder 196 des Schwingungstilgers 191 jeweils beidseitig in die entgegengesetzte Dämpfungsrichtung 198 gedehnt werden. Nach Repulsation des Projektils 111 an dem distalseitigen Anschlagselement 115 bewegt sich das Projektil 111 entgegengesetzt in proximaler Richtung und entsprechend das Gehäuse 104 gegenläufig in distale Richtung 116, wobei die erste Druckfeder 195 und die zweite Druckfeder 196 des Schwingungstilgers 191 komprimiert werden und sich aufeinander zubewegen. Durch die Dehnung oder Kompression der ersten Druckfeder 195 und der zweiten Druckfeder 196 wird zum einen die gegenläufige Bewegung zwischen dem Projektil 111 und dem Gehäuse 104 bei Beschleunigung des Projektils in distaler Richtung 116 oder umgekehrt in proximaler Richtung ausgeglichen und kompensiert, zum anderen werden Vibrationen innerhalb des Gehäuses 104, welche durch den Rückstoß des Projektils 111 jeweils am distalen Anschlagselement 115 oder proximalen Anschlagselement 113 entstehen, mittels der Masse 193 getilgt und mittels der ersten Druckfeder 195 und der zweiten Druckfeder 196 gedämpft.

Somit sind bei Verwendung der Sonotrode 121 zur direkten Zertrümmerung von Körpersteinen sowohl die Schwingungsanregung der Sonotrode 121 mittels des Ultraschallwandlers 171 als auch die Schlaganregung des Projektils 111 mit einer effektiven hohen Zertrümmerungsleistung nutzbar, wobei mittels des Schwingungstilgers 191 durch die Schwingungsanregung und Schlaganregung der Sonotrode 121 induzierte Vibrationen weitgehend reduziert werden. Dadurch kann der der Anwender den Handgriff 103 ruhig und gezielt führen und somit das distale Ende 125 der Sonotrode 121 positionsgenau an dem zu zertrümmernden Körperstein ausrichten kann.

In einer nicht gezeigten Alternative der Lithotripsievorrichtung 101 weist der Handgriff 103 in seinem Gehäuse 104 nicht einen Amplituden-Kompensator 141 auf, sondern stattdessen ist der Schwingungstilger 191 mit seiner ersten Druckfeder 195 direkt an der proximalen Wand des Gegenlagers 177 angeordnet. Hierbei ist der Schwingungstilger 191 direkt auf unerwünschte Schwingungen erzeugt von dem Ultraschallwandler 171 abgestimmt. Ansonsten werden die Lithotripsievorrichtung 101 und der Schwingungstilger 191 wie oben beschrieben betrieben.

In einer in Figur 4 gezeigten Alternative des Schwingungstilgers 191, welche einen Handgriff 103 in Querschnittsdarstellung zeigt, ist die Masse 193 des Schwingungstilgers 191 rohrförmig ausgebildet und konzentrisch um das Beschleunigungsrohr 105 frei von einem Kontakt angeordnet. An der Außenoberfläche 106 der Masse 193 sind mit einem jeweiligen Ende eine erste Druckfeder 195, eine zweite Druckfeder 196 und eine dritte Druckfeder 197 angeordnet, welche mit ihrem gegenüberliegenden Ende jeweils an einer Innenoberfläche 102 des Gehäuses 104 anliegen. Die erste Druckfeder 195, die zweite Druckfeder 196 und die dritte Druckfeder 197 sind in einer Dämpfungsrichtung 198 radial sowohl in Richtung auf die Innenoberfläche 102 des Gehäuses 104 als auch in Richtung auf die Außenoberfläche 106 der Masse 193 beweglich und somit dehnbar und komprimierbar. Bei Betrieb in einer oben beschriebenen Lithotripsievorrichtung 101 schwingt die Masse 193 dreidimensional und radial aufgrund der ersten Druckfeder 195, der zweiten Druckfeder 196 und der dritten Druckfeder 197 um das Beschleunigungsrohr 105, ohne dies zu kontaktieren, wodurch radial umlaufend eine optimale Schwingungstilgung und -dämpfung zu dem umliegenden Gehäuse 104 erzielt wird.

Somit wird ein Schwingungstilger 191 bereitgestellt, welcher je nach den in dem Gehäuse 104 auftretenden Schwingungen und zu vermindernden Vibrationen gezielt mit einer Masse 193 und Federelementen 195, 196, 197 ausbildbar und innerhalb des Gehäuses 104 anordenbar ist.

Die Zeichnungen, die Beschreibung und die Ansprüche enthalten zahlreiche Merkmale in Kombination. Es versteht sich, dass die vorstehend genannten Merkmale nicht nur in der jeweils angegebenen Kombination, sondern auch in anderen Kombinationen oder in Alleinstellung verwendbar sind, ohne den Rahmen der vorliegenden Erfindung zu verlassen. Die Erfindung betrifft eine Haltevorrichtung für eine Lithotripsievorrichtung zum Zertrümmern von Körpersteinen, wobei die Haltevorrichtung ein Gehäuse zum Aufnehmen von Baugruppen und/oder Bauteilen aufweist, und an dem distalen Ende des Gehäuses eine Sonotrode verbindbar ist, wobei in dem Gehäuse ein Beschleunigungsrohr mit einer Längsmittelachse, einem Hohlraum und mit einem bewegbaren Projektil innerhalb des Hohlraums zur Stoßanregung der Sonotrode, ein proximalseitiges Anschlagselement am proximalen Ende und ein distalseitiges Anschlagselement am distalen Ende des Beschleunigungsrohres angeordnet sind, und der Haltevorrichtung eine Krafterzeugungseinrichtung zum Erzeugen einer Kraft zum Hin- und/oder Zurückbewegen des Projektils zuordenbar ist, und in dem Gehäuse eine Schwingungsanregungseinrichtung zur Schwingungsanregung der Sonotrode und eine Schwingungsdämpfungseinrichtung angeordnet sind, wobei die Schwingungsdämpfungseinrichtung mindestens eine Masse und mindestens zwei Federelemente mit jeweils zwei Enden aufweist, wobei die mindestens zwei Federelemente mit jeweils ihrem einen Ende die Masse und mindestens ein Federelement mit seinem zweiten Ende eine Innenoberfläche des Gehäuses kontaktiert. Des Weiteren betrifft die Erfindung eine Lithotripsievorrichtung.

### Bezugszeichenliste

- 101: Lithotripsievorrichtung
- 103: Handgriff
- 102: Innenoberfläche
- 104: Gehäuse
- 105: Beschleunigungsrohr
- 106: Außenoberfläche
- 107: Hohlraum
- 109: proximales Ende
- 110: distales Ende
- 111: Projektil
- 113: proximalseitiges Anschlagselement
- 115: distalseitiges Anschlagselement
- 116: distale Richtung
- 117: Längsmittelachse
- 119: Sonotrodenkopfes
- 121: Sonotrode
- 123: proximales Ende der Sonotrode
- 125: distales Ende der Sonotrode
- 127: Horn
- 129: Hülse
- 131: Deckel
- 133: Rohraufnahme
- 135: elektrischer Anschluss
- 137: Verbindungsstutzen
- 141: Amplituden-Kompensator
- 143: Masseteil
- 145: Feder-Rohrabschnitt
- 147: Verbindungsabschnitt
- 151: Aussparung
- 153: Druckluftreservoir
- 155: Distaler O-Ring
- 157: Proximaler O-Ring
- 159: Kunststoff-Stift
- 171: Ultraschallwandler
- 173: Piezoelement
- 174: elektrischer Kontakt
- 175: Zwischenscheibe
- 176: Hohlbolzen
- 177: Gegenlager
- 179: Proximales Ende des Ultraschallwandlers
- 181: O-Ring
- 183: Platinenhalter
- 187: Druckluftkanal
- 191: Schwingungstilger
- 193: Masse
- 195: erste Druckfeder
- 196: zweite Druckfeder
- 197: dritte Druckfeder
- 198: Dämpfungssrichtung
- 199: Halterung

## Patentansprüche

1. Haltevorrichtung (103) für eine Lithotripsievorrichtung (101) zum Zertrümmern von Körpersteinen, wobei die Haltevorrichtung (103) ein Gehäuse (104) zum Aufnehmen von Baugruppen und/oder Bauteilen aufweist, und das Gehäuse (104) ein distales Ende und ein proximales Ende aufweist und an dem distalen Ende eine Sonotrode (121) verbindbar ist, wobei in dem Gehäuse (104) ein Beschleunigungsrohr (105) als Baugruppe mit einer Längsmittelachse (117), einem Hohlraum (107), einem proximalen Ende (109), einem distalen Ende (110) und mit einem bewegbaren Projektil (111) innerhalb des Hohlraums (107) zur Stoßanregung der Sonotrode (121), ein proximalseitiges Anschlagselement (113) am proximalen Ende (109) und ein distalseitiges Anschlagselement (115) am distalen Ende (110) des Beschleunigungsrohres (105) angeordnet sind, und der Haltevorrichtung (103) eine Krafterzeugungseinrichtung zum Erzeugen einer Kraft zum Hin- und/oder Zurückbewegen des Projektils (111) zwischen dem proximalseitigen Anschlagselement (113) und dem distalseitigen Anschlagselement (115) zuordenbar ist, und in dem Gehäuse (104) eine Schwingungsanregungseinrichtung (171) als Baugruppe zur Schwingungsanregung der Sonotrode (121) und eine Schwingungsdämpfungseinrichtung (191) angeordnet sind, **dadurch gekennzeichnet, dass** die Schwingungsdämpfungseinrichtung (191) mindestens eine Masse (193) und mindestens zwei Federelemente (195, 196, 197) mit jeweils zwei Enden aufweist, wobei die mindestens zwei Federelemente (195, 196, 197) mit jeweils ihrem einen Ende die Masse (193) und mindestens ein Federelement (195, 196) mit seinem zweiten Ende eine Innenoberfläche (102) des Gehäuses (104) kontaktiert.

2. Haltevorrichtung (103) nach Anspruch 1, **dadurch gekennzeichnet, dass** die Schwingungsdämpfungseinrichtung (191) ein drittes Federelement (197) und optional weitere Federelemente aufweist.

3. Haltevorrichtung (103) nach Anspruch 1 oder 2, **dadurch gekennzeichnet, dass** eine Baugruppe, mehrere Baugruppen und/oder alle Baugruppen in dem Gehäuse (104) als jeweils eine Masse (193) der Schwingungsdämpfungseinrichtung (191) ausgebildet ist oder sind.

4. Haltevorrichtung (103) nach einem der vorherigen Ansprüche, **dadurch gekennzeichnet, dass** die Schwingungsanregungseinrichtung (171) oder ein Bauteil der Schwingungsanregungseinrichtung als eine Masse (193) der Schwingungsdämpfungseinrichtung (191) ausgebildet ist.

5. Haltevorrichtung (103) nach einem der vorherigen Ansprüche, **dadurch gekennzeichnet, dass** das Beschleunigungsrohr (105) als eine Masse (193) der Schwingungsdämpfungseinrichtung (191) ausgebildet ist.

6. Haltevorrichtung (103) nach einem der vorherigen Ansprüche, **dadurch gekennzeichnet, dass** das Gehäuse (104) einen Platinenhalter (183) aufweist, wobei der Platinenhalter (183) als eine Masse (193) der Schwingungsdämpfungseinrichtung (191) ausgebildet ist.

7. Haltevorrichtung (103) nach einem der vorherigen Ansprüche, **dadurch gekennzeichnet, dass** die beiden Federelemente (195, 196) auf jeweils einer Seite der Masse (193) in Längsrichtung angeordnet und mittels einer Halteeinheit (199) gehalten sind.

8. Haltevorrichtung (103) nach Anspruch 7, **dadurch gekennzeichnet, dass** ein erstes Federelement (195) proximalseitig der Schwingungsanregungseinrichtung (171) und ein zweites Federelement (196) distalseitig des proximalen Endes des Gehäuses (104) angeordnet sind.

9. Haltevorrichtung (103) nach Anspruch 8, **dadurch gekennzeichnet, dass** zwischen der Schwingungsanregungseinrichtung (171) und dem ersten Federelement (195) eine Schwingungskompensationseinrichtung (141) angeordnet ist.

10. Haltevorrichtung (103) nach einem der Ansprüche 1 bis 4, **dadurch gekennzeichnet, dass** die Masse (193) konzentrisch um das Beschleunigungsrohr (105) angeordnet ist, wobei die Federelemente (195, 196, 197) jeweils mit ihrem einen Ende eine Außenoberfläche (106) der Masse (193) und mit ihrem anderen Ende die Innenoberfläche (102) des Gehäuses (104) kontaktieren.

11. Haltevorrichtung (103) nach einem der vorherigen Ansprüche, **dadurch gekennzeichnet, dass** das jeweilige Federelement (195, 196, 197) und/oder die Halteeinheit (199) eine Stoßdämpfereinheit aufweist.

12. Haltevorrichtung (103) nach einem der vorherigen Ansprüche, **dadurch gekennzeichnet, dass** die Haltevorrichtung (103) distalseitig ein Horn (127) und proximalseitig vom Horn (127) einen Bolzen (176) aufweist, wobei das Horn (127) und der Bolzen (176) einen distalen Abschnitt des Beschleunigungsrohrs (105) umgeben, proximalseitig von dem Horn (127) ein Gegenlager (177) an dem Bolzen (176) angeordnet ist und zwischen dem Gegenlager (177) und dem Horn (127) mindestens ein Piezoelement (173) als Schwingungsanreger angeordnet und mechanisch gekoppelt ist, wobei das Horn (127) das distalseitige Anschlagselement (115) aufweist und/oder das Horn (127) mit dem distalseitigen Anschlagselement (115) und/oder der Sonotrode (121) verbindbar ist und das mindestens eine Piezoelement (173) elektrisch mit einem zuordenbaren Ultraschallgenerator verbindbar ist, wobei die Schwingungsdämpfungseinrichtung (191) proximalseitig an und/oder von dem Horn (127), dem Bolzen (176) und/oder dem Gegenlager (177) angeordnet ist.

13. Lithotripsievorrichtung (101), insbesondere intrakorporale Lithotripsievorrichtung, zum Zertrümmern von Körpersteinen, wobei die Lithotripsievorrichtung eine Sonotrode (121) und eine Haltevorrichtung (103) aufweist, **dadurch gekennzeichnet, dass** die Haltevorrichtung eine Haltevorrichtung (103) nach einem der Ansprüche 1 bis 12 ist.

## Claims

1. A holding device (103) for a lithotripsy device (101) for fragmenting body stones, wherein the holding device (103) has a housing (104) for receiving assemblies and/or components, and the housing (104) has a distal end and a proximal end and a sonotrode (121) can be connected to the distal end, wherein in the housing (104) are arranged an acceleration tube (105) as an assembly with a longitudinal centre axis (117), a cavity (107), a proximal end (109), a distal end (110) and with a movable projectile (111) within the cavity (107) for shock excitation of the sonotrode (121), a proximal-side stop element (113) at the proximal end (109) and a distal-side stop element (115) at the distal end (110) of the acceleration tube (105), and a force generation apparatus for generating a force for moving the projectile (111) back and forth between the proximal-side stop element (113) and the distal-side stop element (115) can be assigned to the holding device (103), and in the housing (104) are arranged a vibration excitation apparatus (171) as an assembly for vibration excitation of the sonotrode (121) and a vibration damping apparatus (191), **characterised in that** the vibration damping apparatus (191) has at least one mass (193) and at least two spring elements (195, 196, 197) each having two ends, wherein the at least two spring elements (195, 196, 197) each contact the mass (193) with their respective one end and at least one spring element (195, 196) contacts an inner surface (102) of the housing (104) with its second end.

2. The holding device (103) according to claim 1, **characterised in that** the vibration damping apparatus (191) has a third spring element (197) and optionally further spring elements.

3. The holding device (103) according to claim 1 or 2, **characterised in that** one assembly, a plurality of assemblies and/or all assemblies in the housing (104) is or are each designed as a mass (193) of the vibration damping apparatus (191).

4. The holding device (103) according to one of the preceding claims, **characterised in that** the vibration excitation apparatus (171) or a component of the vibration excitation apparatus is designed as a mass (193) of the vibration damping apparatus (191).

5. The holding device (103) according to one of the preceding claims, **characterised in that** the acceleration tube (105) is designed as a mass (193) of the vibration damping apparatus (191).

6. The holding device (103) according to one of the preceding claims, **characterised in that** the housing (104) has a circuit board holder (183), wherein the circuit board holder (183) is designed as a mass (193) of the vibration damping apparatus (191).

7. The holding device (103) according to one of the preceding claims, **characterised in that** the two spring elements (195, 196) are each arranged on one side of the mass (193) in the longitudinal direction and are held by means of a holding unit (199).

8. The holding device (103) according to claim 7, **characterised in that** a first spring element (195) is arranged to the proximal side of the vibration excitation apparatus (171) and a second spring element (196) is arranged to the distal side of the proximal end of the housing (104).

9. The holding device (103) according to claim 8, **characterised in that** a vibration compensation apparatus (141) is arranged between the vibration excitation apparatus (171) and the first spring element (195).

10. The holding device (103) according to one of claims 1 to 4, **characterised in that** the mass (193) is arranged concentrically around the acceleration tube (105), wherein the spring elements (195, 196, 197) each contact an outer surface (106) of the mass (193) with their one end and the inner surface (102) of the housing (104) with their other end.

11. The holding device (103) according to one of the preceding claims, **characterised in that** the respective spring element (195, 196, 197) and/or the holding unit (199) has a shock absorber unit.

12. The holding device (103) according to one of the preceding claims, **characterised in that** the holding device (103) has a horn (127) on the distal side and a bolt (176) on the proximal side of the horn (127), wherein the horn (127) and the bolt (176) surround a distal section of the acceleration tube (105), a counter bearing (177) is arranged on the bolt (176) on the proximal side of the horn (127) and at least one piezo element (173) is arranged and mechanically coupled between the counter bearing (177) and the horn (127) as a vibration exciter, wherein the horn (127) has the distal-side stop element (115) and/or the horn (127) can be connected to the distal-side stop element (115) and/or the sonotrode (121), and the at least one piezo element (173) can be electrically connected to an assignable ultrasound generator, wherein the vibration damping apparatus (191) is arranged on the proximal side on and/or of the horn (127), the bolt (176) and/or the counter bearing (177).

13. A lithotripsy device (101), in particular intracorporeal lithotripsy device, for fragmenting body stones, wherein the lithotripsy device comprises a sonotrode (121) and a holding device (103), **characterised in that** the holding device is a holding device (103) according to one of claims 1 to 12.

## Revendications

1. Dispositif de maintien (103) pour un dispositif de lithotripsie (101) destiné à la destruction de calculs corporels, dans lequel le dispositif de maintien (103) présente un boîtier (104) destiné à recevoir des modules et/ou des composants, et le boîtier (104) présente une extrémité distale et une extrémité proximale et une sonotrode (121) peut être reliée à l'extrémité distale, dans lequel un tube d'accélération (105) est monté dans le boîtier (104) en tant que module comportant un axe médian longitudinal (117), une cavité (107), une extrémité proximale (109), une extrémité distale (110) et comportant un projectile mobile (111) à l'intérieur de la cavité (107) destiné à l'excitation par choc de la sonotrode (121), un élément de butée côté proximal (113) est agencé sur l'extrémité proximale (109) et un élément de butée côté distal (115) sur l'extrémité distale (110) du tube d'accélération (105), et un équipement de génération de force destiné à générer une force pour déplacer le projectile (111) dans un sens et/ou dans l'autre entre l'élément de butée côté proximal (113) et l'élément de butée côté distal (115) peut être associé au dispositif de maintien (103), et un équipement d'excitation par vibrations (171) en tant que module destiné à l'excitation par vibrations de la sonotrode (121) et un équipement d'amortissement des vibrations (191) sont agencés dans le boîtier (104), **caractérisé en ce que** l'équipement d'amortissement des vibrations (191) présente au moins une masse (193) et au moins deux éléments élastiques (195, 196, 197) comportant respectivement deux extrémités, dans lequel les au moins deux éléments élastiques (195, 196, 197) sont en contact avec la masse (193) avec respectivement une de leurs extrémités et au moins un élément élastique (195, 196) est en contact avec une surface intérieure (102) du boîtier (104) avec sa seconde extrémité.

2. Dispositif de maintien (103) selon la revendication 1, **caractérisé en ce que** l'équipement d'amortissement des vibrations (191) comporte un troisième élément élastique (197) et, en option, d'autres éléments élastiques.

3. Dispositif de maintien (103) selon la revendication 1 ou 2, **caractérisé en ce qu'**un module, plusieurs modules et/ou tous les modules dans le boîtier (104) est ou sont conçus respectivement comme une masse (193) de l'équipement d'amortissement des vibrations (191).

4. Dispositif de maintien (103) selon l'une des revendications précédentes, **caractérisé en ce que** l'équipement d'excitation par vibrations (171) ou un composant de l'équipement d'excitation par vibrations est conçu comme une masse (193) de l'équipement d'amortissement des vibrations (191).

5. Dispositif de maintien (103) selon l'une des revendications précédentes, **caractérisé en ce que** le tube d'accélération (105) est conçu comme une masse (193) de l'équipement d'amortissement des vibrations (191).

6. Dispositif de maintien (103) selon l'une des revendications précédentes, **caractérisé en ce que** le boîtier (104) présente un support de platine (183), dans lequel le support de platine (183) est conçu comme une masse (193) de l'équipement d'amortissement des vibrations (191).

7. Dispositif de maintien (103) selon l'une des revendications précédentes, **caractérisé en ce que** les deux éléments élastiques (195, 196) sont agencés respectivement d'un côté de la masse (193) dans la direction longitudinale et sont maintenus par le biais d'une unité de maintien (199).

8. Dispositif de maintien (103) selon la revendication 7, **caractérisé en ce qu'**un premier élément élastique (195) est agencé du côté proximal de l'équipement d'excitation par vibrations (171) et un deuxième élément élastique (196) est agencé du côté distal de l'extrémité proximale du boîtier (104).

9. Dispositif de maintien (103) selon la revendication 8, **caractérisé en ce qu'**un équipement de compensation des vibrations (141) est agencé entre l'équipement d'excitation par vibrations (171) et le premier élément élastique (195).

10. Dispositif de maintien (103) selon l'une des revendications 1 à 4, **caractérisé en ce que** la masse (193) est agencée de manière concentrique autour du tube d'accélération (105), dans lequel les éléments élastiques (195, 196, 197) sont respectivement en contact par une de leurs extrémités avec une surface extérieure (106) de la masse (193) et par leur autre extrémité avec la surface intérieure (102) du boîtier (104).

11. Dispositif de maintien (103) selon l'une des revendications précédentes, **caractérisé en ce que** l'élément élastique (195, 196, 197) respectif et/ou l'unité de maintien (199) présente une unité d'amortissement des chocs.

12. Dispositif de maintien (103) selon l'une des revendications précédentes, **caractérisé en ce que** le dispositif de maintien (103) présente, du côté distal, une corne (127) et, du côté proximal de la corne (127), un boulon (176), dans lequel la corne (127) et le boulon (176) entourent une section distale du tube d'accélération (105), un contre-palier (177) est agencé sur le boulon (176) du côté proximal de la corne (127) et au moins un élément piézoélectrique (173) est agencé et accouplé mécaniquement entre le contre-palier (177) et la corne (127) en guise d'excitateur par vibrations, dans lequel la corne (127) présente l'élément de butée côté distal (115) et/ou la corne (127) peut être reliée à l'élément de butée côté distal (115) et/ou à la sonotrode (121) et l'au moins un élément piézoélectrique (173) peut être relié électriquement à un générateur d'ultrasons pouvant être affecté, dans lequel le dispositif d'amortissement des vibrations (191) est agencé du côté proximal sur et/ou à partir de la corne (127), du boulon (176) et/ou du contre-palier (177).

13. Dispositif de lithotripsie (101), en particulier dispositif de lithotripsie intracorporelle, destiné à la destruction de calculs corporels, dans lequel le dispositif de lithotripsie présente une sonotrode (121) et un dispositif de maintien (103), **caractérisé en ce que** le dispositif de maintien est un dispositif de maintien (103) selon l'une des revendications 1 à 12.
